# EUROPEAN PATENT SPECIFICATION

(11) **EP 4 410 786 B1**
(45) Date of publication and mention of the grant of the patent: **09.12.2026**
(21) Application number: 22872194.0
(22) Date of filing: 26.09.2022
(51) Int. Cl.: C07D 401/12, A61P 11/00, A61P 11/06, A61P 11/14, A61P 25/04, A61K 31/53

(54) **3-(3-(4-CHLOROBENZYL)-2,6-DIOXO-4-(4-(PYRIDIN-2-YLOXY)PHENYL)AMINO)-3,6-DIHYDRO-1,3,5-TRIAZIN-1(2H)-YL)-N-(SULFONYL)PROPIONAMIDE DERIVATIVES AS P2X2 AND P2X3 ANTAGONISTS FOR THE TREATMENT OF COUGH, ASTHMA, PAIN OR SLEEP APNEA**
3-(3-(4-CHLORBENZYL)-2,6-DIOXO-4-(4-(PYRIDIN-2-YLOXY)PHENYL)AMINO)-3,6-DIHYDRO-1,3,5-TRIAZIN-1(2H)-YL)-N-(SULFONYL)PROPIONAMID-DERIVATE ALS P2X2- UND P2X3-ANTAGONISTEN ZUR BEHANDLUNG VON HUSTEN, ASTHMA, SCHMERZEN ODER SCHLAFAPNOE
DÉRIVÉS DE 3-(3-(4-CHLOROBENZYL)-2,6-DIOXO-4-(4-(PYRIDIN-2-YLOXY)PHÉNYL)AMINO)-3,6-DIHYDRO-1,3,5-TRIAZIN-1(2H)-YL)-N-(SULFONYL)PROPIONAMIDE COMME ANTAGONISTES P2X2 ET P2X3 POUR LE TRAITEMENT DE LA TOUX, DE L'ASTHME, DE LA DOULEUR OU DE L'APNÉE DU SOMMEIL

(30) Priority: 27.09.2021 CN 202111135636
(43) Date of publication of application: 07.08.2024
(73) Proprietor: Chengdu Shibeikang Biomedical Technology Co., Ltd., Chengdu, Sichuan (CN)
(72) Inventor: ZENG, Yanqun, Chengdu, Sichuan 611730 (CN); ZHU, Xucheng, Chengdu, Sichuan 611730 (CN); HUANG, Long, Chengdu, Sichuan 611730 (CN); ZHU, Tao, Chengdu, Sichuan 611730 (CN); MOU, Xia, Chengdu, Sichuan 611730 (CN); FU, Haixia, Chengdu, Sichuan 611730 (CN)
(74) Representative: Herrero & Asociados, S.L.
(86) International application number: PCT/CN2022/121271
(87) International publication number: WO 2023/046150

(56) References cited:
- WO-A1-2008/040652
- WO-A1-2012/020749
- WO-A1-2013/089212
- WO-A1-2021/054421
- CN-A- 113 773 300

## Description

This application claims priority from the Chinese patent application No. 202111135636.3 filed with the Chinese Patent Office on September 27, 2021, titled "Sulfonamides, Method for Preparation Thereof, and Use Thereof".

### Technical Field

The present invention belongs to the technical field of medicinal chemistry, and specifically relates to sulfonamides, methods for preparation thereof, and uses thereof.

### Background Art

Chronic cough has a high prevalence, with 5% to 10% of adults worldwide suffering from chronic cough, and patients with chronic cough lasting for 8 weeks or more account for more than 1/3 of respiratory outpatient visits, with a prevalence of 2-10%. There are various causes of chronic cough, such as genetic factors, long-term smoking, dietary habits, environmental pollution, cold air, and the like. Chronic cough not only increases the healthcare burden, but also seriously affects the quality of life of patients, causing serious psychological problems. Current treatment of chronic cough generally includes glucocorticoids, β2 receptor agonists, antihistamines, anti-reflux drugs, antibiotics, etc. However, there is currently no approved medicament specifically for chronic cough.

In recent years, it has been found that P2X3 receptors are associated with a variety of disorders, including chronic cough. P2X3 receptors, belonging to the purinergic receptor family, are non-selective ligand-gated ion channels that play an important role in the generation and transmission of injurious messages. Studies suggest that cough reflex hypersensitivity may be mediated by P2X3 receptor specificity. Neuronal hypersensitivity in the airway and lungs caused by an injury or infection may cause excessive, persistent, and frequent coughing.

Gefapixant (MK-7264) is an oral, selective P2X3 receptor antagonist developed by Merck & Co. for the treatment of refractory chronic cough (RCC) or unexplained chronic cough (UCC) in adult patients. A New Drug Application (NDA) for this drug was filed with FDA. Two clinical Phase III trials of the drug demonstrated a statistically significant reduction in the 24-hour cough frequency (the number of coughs per hour measured objectively using 24-hour audio recordings) at Week 12 (the COUGH-1 study) and Week 24 (the COUGH-2 study) in the gefapixant treatment group receiving a dose of 45 mg twice daily, as compared with the placebo group. In these studies, a twice-daily 15-mg dose of gefapixant did not meet the primary endpoint; while the 45-mg group met the clinical endpoints, it showed a higher frequency of discontinuation due to adverse events and a higher incidence of taste-related adverse events. Therefore, there is an urgent demand in the art for a safer and more effective P2X3 receptor antagonist.

WO2021/054421A1 discloses a crystal of a 1,3,5-triazine derivative or a solvate thereof. WO2012/020749A1 discloses a triazine derivative having a P2X3 and/or P2X2/3 receptor antagonistic effect.

### Summary of the invention

An objective of the present invention is to provide a group of sulfonamides having an improved P2X3 receptor antagonizing effect and improved safety.

Another objective of the present invention is to provide a method for preparing the sulfonamides.

Yet another objective of the present invention is to provide use of the sulfonamides.

In order to achieve the above objectives, the technical solutions according to the present invention are provided as follows.

The present invention provides a compound having a structure represented by Formula I, or a pharmaceutically acceptable salt, solvate, or nitrogen-oxide thereof, 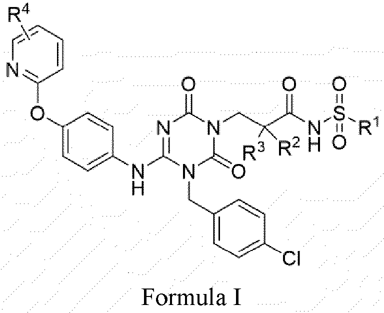
wherein R¹ is selected from a substituted or unsubstituted C1 to C12 alkyl, a substituted or unsubstituted C1 to C12 cycloalkyl, a substituted or unsubstituted C6 to C10 aryl, a substituted or unsubstituted C1 to C12 alkylamino group, and a substituted or unsubstituted C4 to C8 cycloalkylamino group;
R² and R³ are independently selected from hydrogen, halogen, a substituted or unsubstituted C1 to C12 alkyl, and a substituted or unsubstituted C1 to C12 cycloalkyl; or R² and R³ are joined together to form a substituted or unsubstituted 3-membered to 15-membered cycloalkyl group; and
R⁴ is selected from hydrogen or one or two of halogens, methyl, difluoromethyl, trifluoromethyl, difluoromethoxy, and trifluoromethoxy.

In some embodiments according to the present invention, R² and R³ above are independently selected from hydrogen, methyl, ethyl, propyl, isopropyl, and cyclopropyl;
or R² and R³ are joined together to form a cyclopropyl group, a cyclopentyl group, or a cyclohexyl group;
and/or R¹ is selected from a substituted or unsubstituted C1 to C6 alkyl, a substituted or unsubstituted C6 to C10 aryl, and a substituted or unsubstituted C1 to C6 alkylamino group; wherein the substituents on R¹ are one or more selected from deuterium, halogen, hydroxyl, alkyl, alkenyl, alkynyl, alkoxy, alkylamino, alkylthio, amido, NO₂, CN, and CF₃.

In some embodiments according to the present invention, R² and R³ above are independently selected from hydrogen, methyl, ethyl, propyl, isopropyl, and cyclopropyl;
or R² and R³ are joined together to form a cyclopropyl group;
and/or R¹ is selected from methylamino, ethylamino, propylamino, isopropylamino, dimethylamino, tetrahydropyrrolyl, diethylamino, methyl, ethyl, propyl, isopropyl, trifluoromethyl, difluoromethyl, phenyl, halogenated phenyl, tolyl, halogenated tolyl, and halogenated methyloxyphenyl.

In some embodiments according to the present invention, R² and R³ above are independently selected from hydrogen, methyl, and cyclopropyl; or R² and R³ are joined together to form a cyclopropyl group;
and/or R¹ is selected from methyl, ethyl, methylamino, dimethylamino, isopropylamino, tetrahydropyrrolyl, diethylamino, trifluoromethyl, phenyl, tolyl, and fluorophenyl.

In some embodiments according to the present invention, provided are compounds selected from the compounds shown in Table 1 below, or pharmaceutically acceptable salts thereof.

**Table 1**

| | | |
|---|---|---|
| 1 | 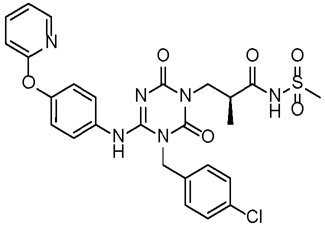 | (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(methanesulfonyl)propionamide |
| 2 | 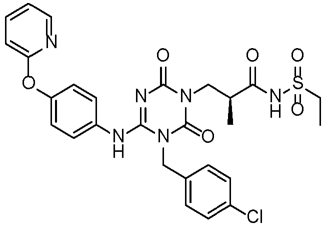 | (S)-3-(3-(4-chlorobenzyl)-2,6-dioxo-4-(4-(pyridin-2-yloxy)phenyl)amino)-3,6-dihydro-1,3,5-triazin-1(2H)-yl)-N-(ethanesulfonyl)-2-methylpropionamide |
| 3 | 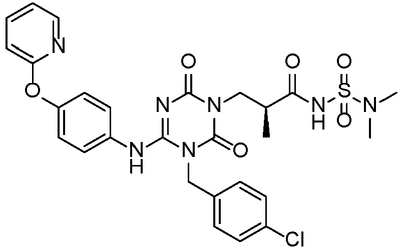 | (S)-3-(3-(4-chlorobenzyl)-2,6-dioxo-4-(4-(pyridin-2-yloxy)phenyl)amino)-3,6-dihydro-1,3,5-triazin-1(2H)-yl)-N-(N,N-dimethylaminosulfonyl)-2-methylpropionamide |
| 4 | 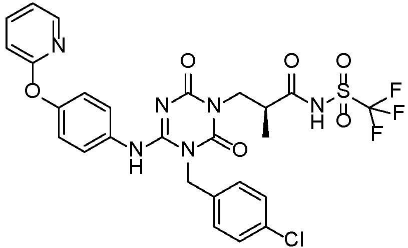 | (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-((trifluoromethyl)sulfonyl)propionamide |
| 5 | 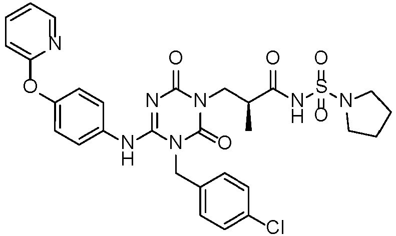 | (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(pyrrolidin-1-ylsulfonyl)propionamide |
| 6 | 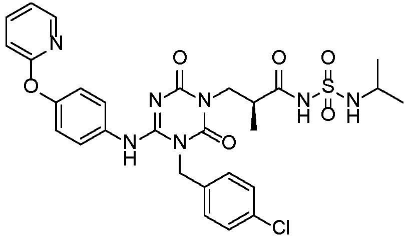 | (S)-3-(3-(4-chlorobenzyl)-2,6-dioxo-4-(4-(pyridin-2-yloxy)phenyl)amino)-3,6-dihydro-1,3,5-triazin-1(2H)-yl)-N-(N-isopropylaminosulfonyl)-2-methylpropionamide |
| 7 | 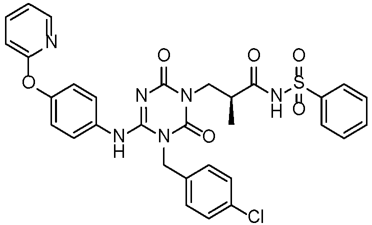 | (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(benzenesulfonyl)propionamide |
| 8 | 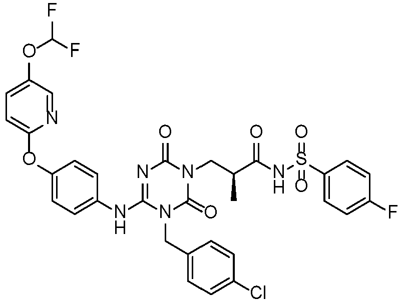 | (S)-3-(3-(4-chlorobenzyl)-4-(4-(5-(5-(difluoromethoxy)pyridin-2-yl)oxyphenyl)amino)-2,6-dioxo-3,6-dihydro-1,3,5-triazin-1(2H)-yl)-N-(4-(4-fluorophenyl)sulfonyl)-2-methylpropionamide |
| 9 | 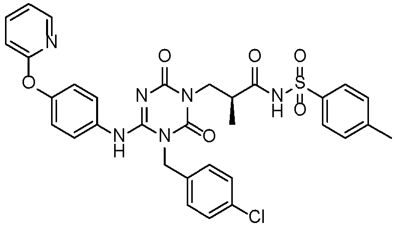 | (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-((4-methylphenyl)sulfonyl)propionamide |
| 10 | 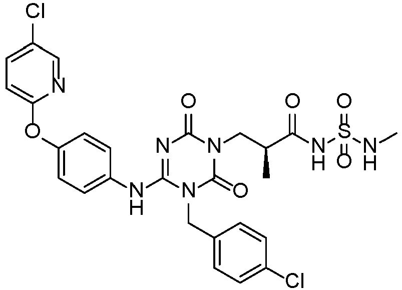 | (S)-3-(3-(4-chlorobenzyl)-4-(4-(5-chloropyridin-2-yloxy)phenyl)amino)-2,6-dioxo-3,6-dihydro-1,3,5-triazin-1(2H)-yl)-2-methyl-N-(N-methylaminosulfonyl)propionamide |
| 11 | 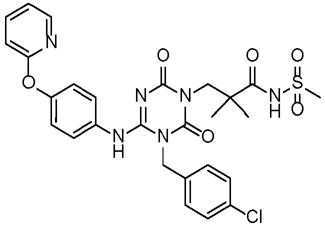 | 3-(3-(4-chlorobenzyl)-2,6-dioxo-4-(4-(pyridin-2-yloxy)phenyl)amino)-3,6-dihydro-1,3,5-triazin-1(2H)-yl)-2,2-dimethyl-N-(methanesulfonyl)propionamide |
| 12 | 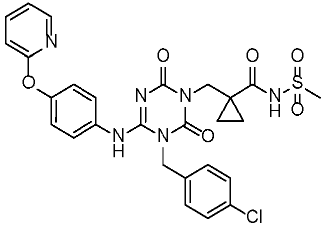 | 1-(3-(4-chlorobenzyl)-2,6-dioxo-4-(4-(pyridin-2-yloxy)phenyl)amino)-3,6-dihydro-1,3,5-triazin-1(2H)-yl)methyl)-N-(methanesulfonyl)cyclopropane-1-carboxamide |

In some embodiments according to the present invention, the hydrogen atoms in the above compounds are replaced by one or more deuterium atoms.

The present invention also provides a method for preparing the above compounds of Formula I, or a salt, solvate, and nitrogen-oxide thereof, comprising the following steps:
Step 1: allowing Compound **a** and Compound **k** to undergo a substitution reaction under a basic condition to produce Compound **b;**
Step 2: allowing intermediate Compounds **c** and **d** to undergo a substitution reaction under a basic condition to obtain Compound **e;**
Step 3: allowing intermediate Compounds **e** and **f** to undergo a Mitsunobu reaction to obtain Compound **g;**
Step 4: allowing intermediate Compound **g** and Compound **b** to undergo a coupling reaction in the presence of a catalyst to obtain Compound **h;**
Step 5: allowing Compound **h** to undergo a hydrolysis reaction catalyzed by an inorganic base or an acid to obtain Compound **j;**
Step 6: allowing Compound **j** and Compound m to undergo a condensation reaction to obtain a compound of Formula **I;** 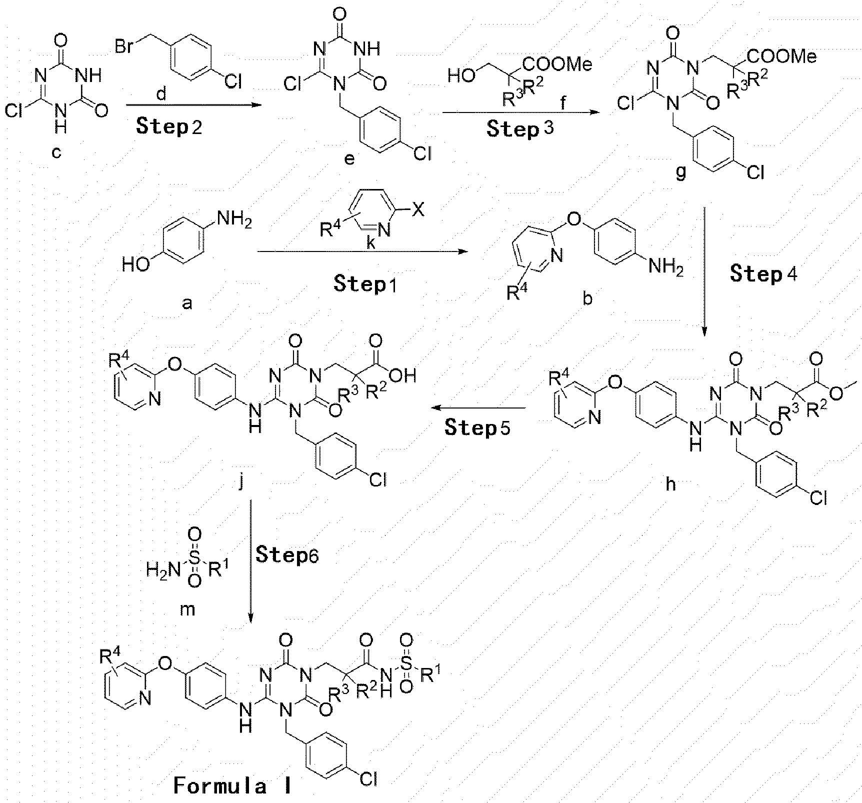
wherein R¹, R², R³, and R⁴ are defined as in any corresponding definitions above, and X is a halogen.

The present invention provides a pharmaceutical formulation comprising the compound of Formula I above and a pharmaceutically acceptable carrier.

The present invention also provides the use of the compound of Formula I above or a salt, solvate, or nitrogen-oxide thereof in the manufacture of a medicament for the treatment or prevention of P2X3 and/or P2X2/3 receptor-associated diseases.

In some embodiments according to the present invention, the above use includes the use of the compound of Formula I above or a salt, solvate, or nitrogen-oxide thereof in the manufacture of a medicament for the treatment or prevention of respiratory disorders, preferably for the treatment or prevention of cough, asthma, pain, or sleep apnea.

The term "pharmaceutically acceptable carrier" used herein refers to a diluent, auxiliary, excipient, or medium which is administered together with a therapeutic agent and is suitable, within reasonable medical judgment, for contact with the tissue of human and/or other animals without causing excessive toxicity, irritation, allergic reactions, or other problems or complications with a reasonable benefit/risk ratio.

The term "halogen" used herein refers to fluorine, chlorine, bromine, and iodine.

As compared with the prior art, the present invention has the following beneficial effects.

The sulfonamides provided according to the present invention have an excellent P2X3 receptor antagonizing effect and safety. The experiments demonstrate that the sulfonamides according to the present invention almost did not affect the sense of taste of mice after intravenous administration at 10 mg/kg, and showed a statistically significant difference from the positive control Gefapixant, indicating that the compounds of the present invention have better safety.

The number of ammonia-induced coughs in mice 30 min after administration of the compounds of the present invention was significantly smaller than that of the positive control, the duration of the cough-relieving effect was significantly prolonged compared with that of the compound of Comparative Example 1, and the inhibitory activity against P2X3 was better than that of the compound of Comparative Example 1 and the positive control Gefapixant, indicating that the compounds of the present invention have a better antagonistic effect against the P2X3 receptor.

The method for preparing the compound of Formula I provided according to the present invention is easy to operate, starts from readily available raw materials, and eases its industrialization.

### Detailed Description of the Invention

The present invention will be described in further detail hereinafter in connection with Examples and experimental examples, which are only used to illustrate the technical solutions of the present invention.

Structures of the compounds were determined by nuclear magnetic resonance (¹H NMR) or liquid mass spectrometry (LC-MS).

The liquid mass spectrometer (LC-MS) was Agilent G6120B (coupled with liquid phase Agilent 1260); the nuclear magnetic resonance (¹H NMR) instrument was Bruker AVANCE-400 or Bruker AVANCE-800, and the nuclear magnetic resonance (¹H NMR) shifts (δ) were given in parts per million (ppm), the solvent for the assay was DMSO, the internal standard was tetramethylsilane (TMS), and the chemical shifts were given in a unit of 10⁻⁶ (ppm).

The term "room temperature" used herein refers to a temperature of 10°C to 25°C.

### Example 1 Preparation of Compound 1:

### (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(methanesulfonyl)propionamide:

### Step 1: Preparation of 4-(pyridin-2-yloxy)aniline (Compound b-1)

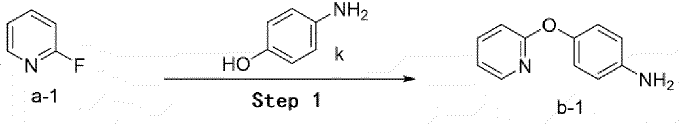

2-Fluoropyridine (97.1 g, 1.0 mol) and p-aminophenol (108.1 g, 0.99 mol) were dissolved in dimethyl sulfoxide (600 ml), to which cesium carbonate (620 g, 1.96 mol) was added to obtain a reaction mixture, which was stirred at a constant rate by mechanical stirring. Subsequently, the reaction system was heated to 80°C to allow a reaction to proceed for 3 h. The reaction progress was tracked by thin-layer chromatography, and when the reaction was complete, the reaction mixture was added to 2 L water under stirring. Afterwards, the product was extracted with ethyl acetate three times, and the extracts were combined and concentrated after removal of ethyl acetate, to obtain a crude product, which was pulped with 500 ml water for 1 h, filtered, and dried in an air drying box, to obtain 4-(pyridin-2-yloxy)aniline (179.7 g, brown granular solid) in 97.8% yield.

ESI-MS: *m*/*z* = 187.1 (M+H)⁺.

### Step 2: Preparation of 6-chloro-1-(4-chlorobenzyl)-1,3,5-triazine-2,4(1H,3H)-dione (compound e)

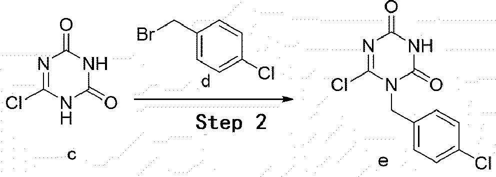

6-Chloro-1,3,5-triazine-2,4(1H,3H)-dione (147.5 g, 1.0 mol) and 4-chlorobenzyl bromide (226.5 g, 1.1 mol) were mixed and dissolved in 300 ml DMF, and then DIPEA (387.6 g, 3.0 mol) was dropwise added there to allow a reaction to proceed at 30°C for 5 h. The reaction progress was tracked by thin-layer chromatography. After the reaction was complete, the reaction mixture was added to 1,000 ml water, and the solid was washed and filtered out. After drying, the filter cake was pulped with 1,000 ml ethyl acetate, and filtered to obtain a solid product which was dried in an air dryer to obtain 6-chloro-1-(4-chlorobenzyl)-1,3,5-triazine-2,4(1H,3H)-dione (compound e) (212.5 g, white solid), yield 78.1%, purity 99.52%.

ESI-MS:m/z=272.0 (M+H)⁺.

### Step 3: Preparation of methyl 3-(4-chloro-3-(4-chlorobenzyl)-2,6-dioxo-3,6-dihydro-1,3,5-triazin-1(2H)-yl)-2-methylpropionate (compound g-1)

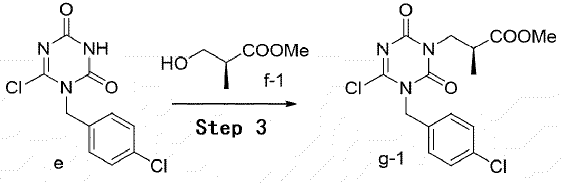

Compound **e** (27.2 g, 0.1 mol), methyl (S)-(+)-3-hydroxy-2-methylpropionate (11.8 g, 0.1 mol), and triphenylphosphine (52.4 g, 0.2 mol) were dissolved in 300 ml anhydrous tetrahydrofuran until clarity. After displacing the air in the reaction system with argon, the reaction system was cooled in an ice-water bath, and diisopropyl azodiformate (40.4 g, 0.2 mol) was added dropwise at a slow and uniform rate under stirring over 30 min. Then a reaction was allowed to proceed while kept at room temperature, and the reaction process was tracked by thin layer chromatography. After the reaction was complete, the reaction solution was quenched with 500 ml water, and extracted with 30 ml ethyl acetate three times, and the organic phase was dried and then concentrated to obtain an oily crude product. The oily crude product was dispersed with a mixed solvent of 100 ml ethyl acetate and 500 ml petroleum ether to precipitate a large amount of triphenylphosphine oxide solid, which was removed by filtration, and the mother liquor was concentrated and purified by chromatography to obtain methyl 3-(4-chloro-3-(4-chlorobenzyl)-2,6-dioxo-3,6-dihydro-1,3,5-triazin-1(2H)-yl)-2-methylpropionate (30.9 g, white solid), yield 83.1%, purity 99.11%.

ESI-MS:m/z=372.1 (M+H)⁺

### Step 4: Preparation of methyl 3-(3-(4-chlorobenzyl)-2,6-dioxo-4-(4-(pyridin-2-yloxy)phenyl)amino)-3,6-dihydro-1,3,5-triazin-1(2h)-yl)-2-methylpropionate (compound h-1)

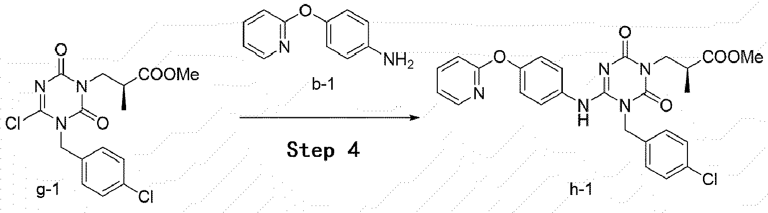

Compound **g-1** (3.72 g, 0.01 mol), Compound **b-1** (1.87 g, 0.01 mol), xant-phos (868 mg, 1.5 mmol), palladium acetate (337 mg, 1.5 mmol), and potassium phosphate (4.24 g, 0.02 mol) were mixed and dissolved in 30 ml dioxane, and the reaction flask was purged with argon. The reaction mixture was heated in an oil bath to 80°C to allow a reaction to proceed for 1-2 h under argon protection until Compound **g-1** was completely consumed as monitored by thin layer chromatography. The reaction mixture was distilled under reduced pressure to remove dioxane, and was extracted with 100 ml ethyl acetate and with 100 ml water three times. The ethyl acetate phase was dried, concentrated, and purified by column chromatography to obtain methyl 3-(3-(4-chlorobenzyl)-2,6-dioxo-4-(4-(pyridin-2-yloxy)phenyl)amino)-3,6-dihydro-1,3,5-triazin-1(2h)-yl)-2-methylpropionate (4.60 g, as yellowish brown foamy solid), yield 88.1%, purity 97.34%.

ESI-MS:m/z=522.2 (M+H)⁺.

¹H NMR (400 MHz, DMSO-d6) δ 8.63 (s, 1H), 8.20-8.11 (m, 1H), 7.90-7.79 (m, 1H), 7.48-7.38 (m, 2H), 7.29 (d, J = 8.3 Hz, 2H), 7.21-7.14 (m, 4H), 7.14-7.10 (m, 1H), 7.03 (d, J = 8.3 Hz, 1H), 5.28 (s, 2H), 3.89 (m, 2H), 3.47 (s, 3H), 2.77 (m, 1H), 1.00 (m, 3H).

### Step 5: Preparation of (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methylpropanoic acid (compound j-1)

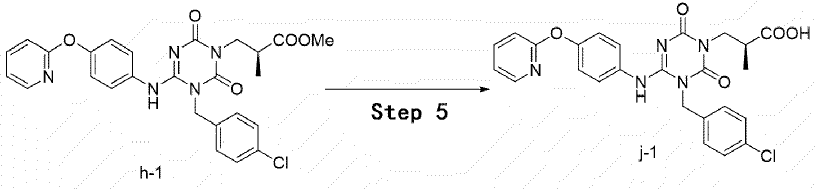

Compound **h-1** (522 mg, 1.0 mmol) was dissolved in a mixed solvent of methanol (3 ml) and tetrahydrofuran (3 ml) kept at about 10°C, and a solution of lithium hydroxide (168 mg, 4 mmol) in water (3 ml) was added thereto to obtain a reaction mixture, which was allowed to react at room temperature overnight. The reaction progress was tracked by thin layer chromatography. After the reaction was complete, it was purified by column chromatography to obtain (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methylpropanoic acid (409 mg, off-white solid); yield 80.5%; purity of 99.69%.

ESI-MS: *m*/*z* = 508.1(M+H) ⁺.

¹H NMR (400 MHz, DMSO-d6) δ 8.16 (dd, J = 5.1, 2.0 Hz, 1H), 7.94-7.80 (m, 1H), 7.48-7.39 (m, 2H), 7.35-7.26 (m, 2H), 7.16 (m, 4H), 7.14-7.11 (m, 1H), 7.04 (d, J = 8.3 Hz, 1H), 5.30 (s, 2H), 4.06-3.79 (m, 2H), 2.75 (m, 1H), 0.98 (m, 3H).

### Step 6: Preparation of (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(methanesulfonyl)propionamide (Compound 1)

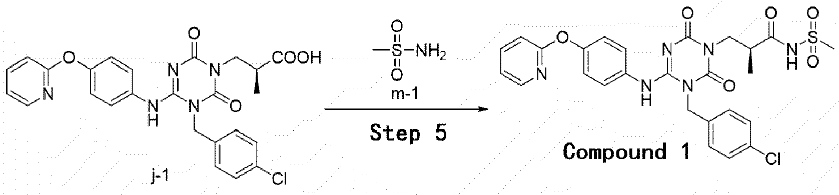

Compound **j-1** (254 mg, 0.5 mmol) and N,N'-carbonyldiimidazole (CDI) (89 mg, 0.55 mmol) were dissolved in 5 mL anhydrous THF, and heated to reflux for 1 h, followed by cooling. Then methanesulfonamide (47.5 mg, 0.5 mmol) was added and stirred for 1 h, and DBU (0.125 mL, 0.5 mmol) was added dropwise, to allow a reaction to proceed at room temperature overnight. The reaction solution was poured into 1N HCl and extracted with ethyl acetate. The mixed organic phase was washed with water and saturated brine, dried over MgSO₄, and filtered, followed by solvent removal under reduced pressure, purification by silica gel chromatography, collection under reduced pressure, and vacuum drying, to obtain (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(methanesulfonyl)propionamide (197.7 mg) yield 67.6%, purity 99.85%.

ESI-MS: *m*/*z* = 585.1 (M+H) ⁺.

¹H NMR (400 MHz, DMSO-d6) δ 11.69 (s, 1H), 8.60 (s, 1H), 8.15 (dd, J = 5.0, 2.0 Hz, 1H), 7.90-7.79 (m, 1H), 7.45-7.37 (m, 2H), 7.30 (d, J = 8.4 Hz, 2H), 7.15 (m, 4H), 7.14-7.09 (m, 1H), 7.03 (d, J = 8.3 Hz, 1H), 5.42-5.15 (m, 2H), 3.88 (m, 2H), 3.01 (s, 3H), 2.69 (m, 1H), 0.98 (m, 3H).

### Example 2 Preparation of Compound 2:

### (S)-3-(3-(4-chlorobenzyl)-2,6-dioxo-4-(4-(pyridin-2-yloxy)phenyl)amino)-3,6-dihydro-1,3,5-triazin-1(2H)-yl)-N-(ethanesulfonyl)-2-methylpropionamide

The method for preparation in this Example was the same as that in Example 1 except that Compound m-1 methanesulfonamide in Step 6 was replaced with equimolar ethanesulfonamide, to obtain (S)-3-(3-(4-chlorobenzyl)-2,6-dioxo-4-(4-(pyridin-2-yloxy)phenyl)amino)-3,6-dihydro-1,3,5-triazin-1(2H)-yl)-N-(ethylsulfonyl)-2-methylpropionamide (Compound 2) as a white solid, yield: 77.0%, purity 99.13%.

ESI-MS: *m*/*z* = 599.2 (M+H)⁺.

¹H NMR (400 MHz, DMSO-d6) δ 11.53 (s, 1H), 8.60 (s, 1H), 8.15 (dd, J = 5.0, 2.0 Hz, 1H), 7.90-7.79 (m, 1H), 7.45-7.37 (m, 2H), 7.30 (d, J = 8.4 Hz, 2H), 7.15 (m, 4H), 7.14-7.09 (m, 1H), 7.03 (d, J = 8.3 Hz, 1H), 5.42-5.15 (m, 2H), 3.88 (m, 2H), 3.33 (m, 2H), 2.69 (m, 1H), 1.27 (m, 3H),0.98 (m, 3H).

### Example 3 Preparation of Compound 3:

### (S)-3-(3-(4-chlorobenzyl)-2,6-dioxo-4-(4-(pyridin-2-yloxy)phenyl)amino)-3,6-dihydro-1,3,5-triazin-1(2H)-yl)-N-(N,N-dimethylaminosulfonyl)-2-methylpropionamide

The method for preparation in this Example was the same as that in Example 1 except that Compound **m-1** methanesulfonamide in Step 6 was replaced with equimolar N,N-dimethylaminosulfonamide, to obtain (S)-3-(3-(4-chlorobenzyl)-2,6-dioxo-4-(4-(pyridin-2-yloxy)phenyl)amino)-3,6-dihydro-1,3,5-triazin-1(2H)-yl)-N-(N,N-dimethylaminosulfonyl)-2-methylpropionamide (Compound **3)** as a white solid, yield: 78.6%, purity: 98.12%.

ESI-MS: *m*/*z =* 614.2 (M+H) ⁺.

¹H NMR (400 MHz, DMSO-d6) δ 11.28(s, 1H), 8.60 (s, 1H), 8.15 (dd, J = 5.0, 2.0 Hz, 1H), 7.90-7.79 (m, 1H), 7.45-7.37 (m, 2H), 7.30 (d, J = 8.4 Hz, 2H), 7.15 (m, 4H), 7.14-7.09 (m, 1H), 7.03 (d, J = 8.3 Hz, 1H), 5.42-5.15 (m, 2H), 3.88 (m, 2H), 2.79 (s, 6H), 2.69 (m, 1H), 0.98 (m, 3H).

### Example 4 Preparation of Compound 4:

### (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-((trifluoromethyl)sulfonyl)propionamide

The method for preparation in this Example was the same as that in Example 1 except that Compound **m-1** methanesulfonamide in Step 6 was replaced with equimolar trifluoromethylsulfonamide, to obtain (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-((trifluoromethyl)sulfonyl)propionamide (Compound 4) as a white solid, yield: 76.5%, purity: 99.71%.

ESI-MS: *m*/*z* = 639.2 (M+H)⁺.

¹H NMR (400 MHz, DMSO-d6) δ 11.69 (s, 1H), 8.60 (s, 1H), 8.15 (dd, J = 5.0, 2.0 Hz, 1H), 7.90-7.79 (m, 1H), 7.45-7.37 (m, 2H), 7.30 (d, J = 8.4 Hz, 2H), 7.15 (m, 4H), 7.14-7.09 (m, 1H), 7.03 (d, J = 8.3 Hz, 1H), 5.42-5.15 (m, 2H), 3.88 (m, 2H), 2.69 (m, 1H), 0.98 (m, 3H).

### Example 5 Preparation of Compound 5:

### (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(pyrrolidin-1-ylsulfonyl)propionamide

The method for preparation in this Example was the same as that in Example 1 except that Compound **m-1** methanesulfonamide in Step 6 was replaced with equimolar pyrrolidinylsulfonamide, to obtain (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(pyrrolidin-1-ylsulfonyl)propionamide (Compound **5)** as a white solid, yield: 78.2%, purity: 99.12%.

ESI-MS: *m*/*z* =640.2 (M+H) ⁺.

¹H NMR (400 MHz, DMSO-d6) δ 11.26 (s, 1H), 8.60 (s, 1H), 8.15 (dd, J = 5.0, 2.0 Hz, 1H), 7.90-7.79 (m, 1H), 7.45-7.37 (m, 2H), 7.30 (d, J = 8.4 Hz, 2H), 7.15 (m, 4H), 7.14-7.09 (m, 1H), 7.03 (d, J = 8.3 Hz, 1H), 5.42-5.15 (m, 2H), 3.88 (m, 2H), 3.37-3.60 (m, 4H), 2.69 (m, 1H), 1.76-1.74(m, 4H), 0.98 (m, 3H).

### Example 6 Preparation of Compound 6:

### (S)-3-(3-(4-chlorobenzyl)-2,6-dioxo-4-(4-(pyridin-2-yloxy)phenyl)amino)-3,6-dihydro-1,3,5-triazin-1(2H)-yl)-N-(N-isopropylaminosulfonyl)-2-methylpropionamide

The method for preparation in this Example was the same as that in Example 1 except that Compound **m-1** methanesulfonamide in Step 6 was replaced with equimolar N-isopropylaminosulfonamide, to obtain (S)-3-(3-(4-chlorobenzyl)-2,6-dioxo-4-(4-(pyridin-2-yloxy)phenyl)amino)-3,6-dihydro-1,3,5-triazin-1(2H)-yl)-N-(N-isopropylaminosulfonyl)-2-methylpropionamide (Compound **6)** as a white solid, yield: 76.6%, purity: 97.64%.

ESI-MS: *m*/*z* =628.1 (M+H) ⁺.

¹H NMR (400 MHz, DMSO-d6) δ 11.17 (s, 1H), 8.60 (s, 1H), 8.15 (dd, J = 5.0, 2.0 Hz, 1H), 7.90-7.79 (m, 1H), 7.45-7.37 (m, 3H), 7.30 (d, J = 8.4 Hz, 2H), 7.15 (m, 4H), 7.14-7.09 (m, 1H), 7.03 (d, J = 8.3 Hz, 1H), 5.42-5.15 (m, 2H), 3.88 (m, 2H), 2.83 (m,1H), 2.69 (m, 1H), 1.22-1.22 (d,J=6.6Hz, 6H), 0.98 (m, 3H).

### Example 7 Preparation of Compound 7:

### (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(benzenesulfonyl)propionamide

The method for preparation in this Example was the same as that in Example 1 except that Compound **m-1** methanesulfonamide in Step 6 was replaced with equimolar benzenesulfonamide, to obtain (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-(benzenesulfonyl)propionamide (Compound 7) as a white solid, yield: 74.2%, purity 99.68%.

ESI-MS: *m*/*z* = 647.1 (M+H) ⁺.

¹H NMR (400 MHz, DMSO-d6) δ 12.06 (s, 1H), 8.60 (s, 1H), 8.15 (dd, J = 5.0, 2.0 Hz, 1H) , 7.96-7. 90 (m, 2H), 7.90-7.79 (m, 1H), 7.74-7.58 (m, 3H), 7.45-7.37 (m, 2H), 7.30 (d, J = 8.4 Hz, 2H), 7.15 (m, 4H), 7.14-7.09 (m, 1H), 7.03 (d, J = 8.3 Hz, 1H), 5.42-5.15 (m, 2H), 3.88 (m, 2H), 2.69 (m, 1H), 0.98 (m, 3H).

### Example 8 Preparation of Compound 8:

### (S)-3-(3-(4-chlorobenzyl)-4-(4-(5-(5-(difluoromethoxy)pyridin-2-yl)oxyphenyl)amino)-2,6-dioxo-3,6-dihydro-1,3,5-triazin-1(2H)-yl)-N-(4-(4-fluorophenyl)sulfonyl)-2-methylpropionamide

The method for preparation in this Example was the same as that in Example 1 except that Compound **a-1** 2-fluoropyridine in Step 1 was replaced with equimolar 2-fluoro-5-difluoromethoxypyridine, and Compound **m-1** methanesulfonamide in Step 6 was replaced with equimolar 4-fluorobenzenesulfonamide, to obtain (S)-3-(3-(4-chlorobenzyl)-4-(4-(5-(5-(difluoromethoxy)pyridin-2-yl)oxyphenyl)amino)-2,6-dioxo-3,6-dihydro-1,3,5-triazine-1(2H)-yl)-N-(4-(4-fluorophenyl)sulfonyl)-2-methylpropionamide (Compound **8),** yield: 78.2%, purity 98.98%.

ESI-MS: *m*/*z* = 731.2 (M+H) ⁺.

¹H NMR (400 MHz, DMSO-d6) δ 12.13(s, 1H), 8.59 (s, 1H), 8.02 (s, 1H) , 7.98-7. 93 (m, 2H), 7.82-7.73 (m, 1H), 7.52 (s, 1H), 7.45-7.37 (m, 4H), 7.30 (d, J = 8.4 Hz, 2H), 7.15 (m, 4H), 7.11-7.06 (m, 1H) , 5.42-5.15 (m, 2H), 3.86 (m, 2H), 2.68 m, 1H), 0.98 (m, 3H).

### Example 9 Preparation of Compound 9:

### (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-((4-methylphenyl)sulfonyl)propionamide

The method for preparation in this Example was the same as that in Example 1 except that Compound **m-1** methanesulfonamide in Step 6 was replaced with equimolar 4-methylbenzenesulfonamide, to obtain (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methyl-N-((4-methylphenyl)sulfonyl)propionamide (Compound **9)** as a while solid; yield: 75.2%, purity: 99.11%.

ESI-MS: *m*/*z* = 661.1 (M+H) ⁺.

¹H NMR (400 MHz, DMSO-d6) δ 11.97 (s, 1H), 8.60 (s, 1H), 8.15 (dd, J = 5.0, 2.0 Hz, 1H), 7.90-7.79 (m, 1H), 7.81 (d, J= 8.3 Hz, 2H), 7.45-7.37 (m, 4H), 7.30 (d, J = 8.4 Hz, 2H), 7.15 (m, 4H), 7.14-7.09 (m, 1H), 7.03 (d, J = 8.3 Hz, 1H), 5.42-5.15 (m, 2H), 3.88 (m, 2H), 2.37(s, 3H), 2.69 (m, 1H), 0.98 (m, 3H).

### Example 10 Preparation of Compound 10:

### (S)-3-(3-(4-chlorobenzyl)-4-(4-(5-chloropyridin-2-yloxy)phenyl)amino)-2,6-dioxo-3,6-dihydro-1,3,5-triazin-1(2H)-yl)-2-methyl-N-(N-methylaminosulfonyl)propionamide

The method for preparation in this Example was the same as that in Example 1 except that Compound **a-1** 2-fluoropyridine in Step 1 was replaced with equimolar 2-fluoro-5-chloropyridine, and Compound **m-1** methanesulfonamide in Step 6 was replaced with equimolar methylaminosulfonamide, to obtain (S)-3-(3-(4-chlorobenzyl)-4-(4-(5-chloropyridin-2-yloxy)phenyl)amino)-2,6-dioxo-3,6-dihydro-1,3,5-triazin-1(2H)-yl)-2-methyl-N-(N-methylaminosulfonyl)propionamide (Compound **10)** as a white solid, yield: 73.8%, purity 98.99%.

ESI-MS: *m*/*z =* 521.2 (M+H)⁺.

¹H NMR (400 MHz, DMSO-d6) δ 11.18 (s, 1H), 8.58 (s, 1H), 8.18 (s, 1H), 7.90-7.79 (m, 1H), 7.45-7.37 (m, 3H), 7.30 (d, J = 8.4 Hz, 2H), 7.22 (d, J = 8.3 Hz, 1H), 7.13 (m, 4H), 5.42-5.15 (s, 2H), 3.88 (m, 2H), 2.80 (s, 3H), 2.69 (m, 1H), 0.98 (m, 3H).

### Example 11 Preparation of Compound 11:

### 3-(3-(4-chlorobenzyl)-2,6-dioxo-4-(4-(pyridin-2-yloxy)phenyl)amino)-3,6-dihydro-1,3,5-triazin-1(2H)-yl)-2,2-dimethyl-N-(methanesulfonyl)propionamide

The method for preparation in this Example was the same as that in Example 1 except that Compound **f-1** methyl (S)-3-hydroxy-2-methylpropionate in Step 3 was replaced with equimolar methyl 3-hydroxy-2,2-dimethylpropionate, to obtain 3-(3-(4-chlorobenzyl)-2,6-dioxo-4-(4-(pyridin-2-yloxy)phenyl)amino)-3,6-dihydro-1,3,5-triazin-1(2H)-yl)-2,2-dimethyl-N-(methanesulfonyl)propionamide (Compound **11)** as a white solid, yield: 75.9%, purity: 97.28%.

ESI-MS: *m*/*z* = 599.2 (M+H)⁺.

¹H NMR (400 MHz, DMSO-d6) δ 11.30 (s, 1H), 8.64 (s, 1H), 8.20-8.09 (m, 1H), 7.84 (m, 1H), 7.42 (d, J = 8.6 Hz, 2H), 7.31-7.25 (m, 2H), 7.20-7.13 (m, 4H), 7.13-7.09 (m, 1H), 7.03 (d, J = 8.3 Hz, 1H), 5.25 (s, 2H), 4.03 (s, 2H), 3.02 (s, 3H), 1.05 (s, 6H).

### Example 12 Preparation of Compound 12:

### 1-(3-(4-chlorobenzyl)-2,6-dioxo-4-(4-(pyridin-2-yloxy)phenyl)amino)-3,6-dihydro-1,3,5-triazin-1(2H)-yl)methyl)-N-(methanesulfonyl)cyclopropane-1-carboxamide

The method for preparation in this Example was the same as that in Example 1 except that Compound **f-1** methyl (S)-3-hydroxy-2-methylpropionate in Step 3 was replaced with equimolar methyl 1-(hydroxymethyl)cyclopropane-1-carboxylate, to obtain 1-(3-(4-chlorobenzyl)-2,6-dioxo-4-(4-(pyridin-2-yloxy)phenyl)amino)-3,6-dihydro-1,3,5-triazin-1(2H)yl)methyl)-N-(methanesulfonyl)cyclopropane-1-carboxamide (Compound **12)** as a white solid, yield: 71.8%, purity: 99.11%.

ESI-MS: *m*/*z =* 597.1 (M+H)⁺.

¹H NMR (400 MHz, DMSO-d6) δ 11.32 (s, 1H), 8.64 (s, 1H), 8.15 (dd, J = 5.2, 2.0 Hz, 1H), 7.85 (m, 1H), 7.46-7.38 (m, 2H), 7.31 (d, J = 8.4 Hz, 2H), 7.23-7.15 (m, 4H), 7.15-7.09 (m, 1H), 7.04 (d, J = 8.3 Hz, 1H), 5.28 (s, 2H), 4.13 (s, 2H), 3.12 (s, 3H), 1.07-0.91(m, 4H).

### Comparative Example 1 Preparation of Comparative Compound 1:

### (S)-3-(3-(4-chlorobenzyl)-2,6-dioxo-4-(4-(pyridin-2-yloxy)phenyl)amino)-3,6-dihydro-1,3,5-triazin-1(2H)-yl)-2-methylpropionic acid

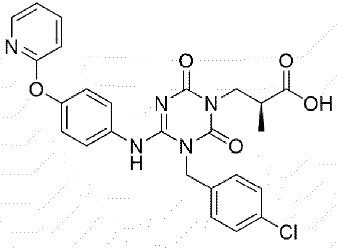

Comparative Example 1 is Compound **j-1** as an intermediate produced during the synthesis of the Examples. It was prepared in the same way as the preparation for Compound j-1 in Example 1.

### Experimental Example 1: Mouse coughing test

### 1 Experimental materials

### 1.1 Basic information of the test materials

Compounds 1 to 12 of Examples 1 to 12 were synthesized in the laboratory by the present inventors; positive control was Gefapixant, Batch No.: 01030-210326-2-1 (purchased from Tianjin Holder Pharmaceutical Technology Co., Ltd.); Comparative Compound 1 was synthesized in the laboratory by the present inventors.

### 1.2 Reagents

### Physiological saline, aqueous ammonia.

### 2 Laboratory animals

Healthy adult KM mice, half male and half female, 6 mice in each group, each weighing about 28-30 g.

### 3 Experimental method

### 3.1 Dosage design and amount of test material

Most of the animal coughing models reported in the literature use mechanical, chemical and electrical stimulation to stimulate the animal's nerves and receptors to trigger coughing. Based on the characteristics of the candidate compounds and the existing similar target compounds as a reference, the concentrated ammonia induction method was preliminarily selected to establish a coughing model in mice.

### 3.2 Preparation of test materials

Preparation of 50% aqueous ammonia solution: 2.5 ml aqueous ammonia was dissolved in a 5 ml injection solution of 0.9% sodium chloride, and mixed well.

Preparation of solutions of the positive control and Comparative Compound 1: each of 9 mg positive control and 9 mg Comparative Compound 1 was weighed and dissolved in a 3 ml solution of 0.5% CMC-Na, and fully mixed, to obtain a 3 mg/ml solution.

Preparation of solutions of the compounds of Examples: 9 mg of the compound of each Example was dissolved in a 3 ml solution of 0.5% CMC-Na, and mixed thoroughly, to obtain a 3 mg/ml solution.

### 3.3 Experimental operation

Grouping: Comparative Example 1 group, Positive control group, Example group, Model group. Six KM mice in each group were intragastrically given Comparative Compound 1 (30 mg/kg), the positive control (Gefapixant, purchased, 30 mg/kg), and an Example compound (30 mg/kg), and the model group was given an equal volume of 0.5% CMC-Na solution. 30 min, 60 min, or 120 min after administration, each mouse was placed in a 500 ml beaker, and a cotton ball (weight: 100±5 mg) containing 0.3 ml 50% aqueous ammonia was placed in the beaker. The number of typical coughs by the mouse within 3 min was observed (typical cough: contraction of the abdominal muscle or contraction of the chest, accompanied by wide opening of the mouth and a coughing sound).

### 4 Results and Discussion

### 4.1 Judgment criteria for results

(1) Cough judgment criteria:
   Cough was judged by contraction of the abdominal muscle or contraction of the chest, accompanied by wide opening of the mouth and a coughing sound.
(2) The number of coughs by the mice within 3 min was recorded with a stop watch, and statistically analyzed with software. The data of each group were statistically expressed in mean ± standard deviation, and one-way ANOVA between multiple groups was performed, P<0.05 for statistically significant difference.

### 4.2 Discussion

4.2.1 The number of coughs by mice 30 min after administration of 30 mg/kg of the example compounds

**Table 2**

| **Groups** | **Number of coughs** | **Groups** | **Number of coughs** |
|---|---|---|---|
| Model group | 51.30 | Example 6 | 20.60** |
| Positive control | 20.51** | Example 7 | 28.66* |
| Comparative Example 1 | 15.61** | Example 8 | 29.83* |
| Example 1 | 13.33** | Example 9 | 25.11* |
| Example 2 | 17.12** | Example 10 | 20.27** |
| Example 3 | 15.34** | Example 11 | 17.62** |
| Example 4 | 28.61* | Example 12 | 19.39** |
| Example 5 | 12.85** | / | / |

| | | | |
|---|---|---|---|
| Note: as compared with the model group: **P<0.01, *P<0.05. | | | |

As can be seen through Table 2, the number of ammonia-induced coughs by the mice in the positive control group 30 min after administration was significantly reduced as compared with that of the model group, with a statistically significant difference (P<0.01), indicating a successful modeling. The number of coughs in the groups of example compounds was significantly reduced as compared with that of the model group, with a statistically significant difference (P<0.01).

4.2.2 Number of coughs 60 and 120 min after administration of test samples at 30 mg/kg

**Table 3**

| **Groups** | **Number of coughs** | |
|---|---|---|
| | 60 min | 120 min |
| Model group | 48.90 | 51.05 |
| Comparative Example 1 | 22.29** | 45.21 |
| Example 1 | 18.37** | 17.28**^{△} |
| Example 3 | 16.98** | 18.56**^{△} |
| Example 5 | 16.25 ** | 23.11**^{△} |
| Example 6 | 17.63** | 28.78*^{△} |
| Example 10 | 20.33** | 24.13**^{△} |
| Example 11 | 16.68** | 18.90**^{△} |
| Example 12 | 20.17** | 24.14**^{△} |

| | | |
|---|---|---|
| Note: as compared with the model group: **P<0.01, *P<0.05; as compared with Comparative Example 1: ^{△}P<0.05. | | |

As can be seen through Table 3, as compared with the model group, the number of coughs 60 min after administration in the Comparative example 1 group was significantly reduced, with a statistically significant difference (P<0.01); the number of coughs 120 min after administration in the Comparative Example 1 group was not statistically different from that of the model group, indicating that the compound of Comparative example 1 group did not have an obvious cough relieving effect 120 min after administration.

Example groups 1, 3, 5, 6, 10, 11, and 12 each showed a significantly reduced number of coughs group as compared with the model group, not only 60 min after administration, but also 120 min after administration, with a statistically significant difference (P<0.05). This indicates that the duration of cough-relieving action of Compounds 1, 3, 5, 6, 10, 11 and 12 of the present invention was significantly prolonged compared to that of Comparative Compound 1.

### Experimental Example 2: In vitro biological activity evaluation

The reagents, consumables, and instrument used in this example were all commercially available.

### 1. Cell line

A HEK293 cell line stably transfected with human P2X3 receptor was used.

### 2. Cell culture

Growth medium: DMEM high glucose;
10% FBS;
1% PenStrep.

### 3. Cell culturing procedure:

### a) Resuscitation of cells

1) A frozen cell tube was immersed in a 37°C water bath, and shaken continuously to thaw as soon as possible;
2) The cells were slowly blown up and down with a 1 mL pipette to be suspended, added dropwise to a 15 mL centrifuge tube containing 10 mL fresh pre-warmed growth medium, and then centrifuged for 5 minutes at 1,000 rpm;
3) The supernatant was discarded and the cells were re-suspended with 5 mL fresh growth medium. The cell suspension was transferred to a petri dish and left in a 5% CO₂ incubator at 37°C for incubation;
4) After 24 hours, the medium was slowly removed (carefully not to destroy the cell monolayer), and incubated with fresh growth medium.

### b) Passaging cells

The cell line was usually passaged at a dilution ratio of 1:3 to 1:4, twice a week (the 1:3 passaging ratio was more frequently used), and it took 2 to 3 days for the passaged cells to grow to 85% confluency;
1) After the cells reached >85% saturation in a 10 cm dish, the dish was digested with a 0.25% Trypsin-EDTA solution for about 1 min, and the cells were aspirated out of the dish;
2) The cells were transferred to a Petri dish containing a complete growth medium according to the dilution ratio. Note: to maintain the exponential growth of cells, the cell monolayer culturing should be maintained;
3) The cells were passaged using a 0.25% trypsin solution according to the cell doubling time of the cell line (HEK293-P2X3: 24 hours).

### c) Cryopreservation of cells

1) The petri dish was removed from the incubator, placed in an ultra-clean bench, and digested with a 0.25% Trypsin-EDTA solution for about 1 min. The cells were collected, counted, and centrifuged for 5 min at 1,000 rpm;
2) The supernatant was aspirated out and the cells were re-suspended in a cryopreservation solution (90% FBS and 10% DMSO) at a density of 2×10⁶ cells/mL, with 1 mL cell suspension in each cryopreservation tube;
3) The cell cryopreservation tubes were placed in a cryopreservation box and transferred to -80°C overnight;
4) The cryopreservation tubes were transferred to a liquid nitrogen tank (-196°C).

### 4. Experimental procedure

### Step 1: Preparation of cell experiment plate

1) When the cells in a 15 cm Petri dish grew to 80% confluency, the supernatant was removed, 5 mL DPBS was added to wash the cells and then aspirated out, then 2.5 mL of a 0.25% Trypsin-EDTA solution was added to the Petri dish, the Petri dish was placed in an incubator for 1 to 3 minutes or until the cells were digested to fall off, then 3 mL of a complete medium was added to terminate the digestion, and the cell density was measured by a cell counter;
2) The cells were centrifuged at 1,000 rpm for 5 min, and re-suspended in growth medium, and the volume of the suspension was adjusted to a cell density of 4×10⁵ cells/mL (1×10⁴ cells/25 µL);
3) 10 µL 5×Matrigel was added to a black microtiter plate, which was placed in an incubator for 15 min, then taken out, inverted and centrifuged at 300 g/min for 30 s to remove the 5×Matrigel; then the prepared cell suspension was added to the 384 black microtiter plate at 25 µL per well;
4) The microtiter plate was placed in a 5% CO₂ incubator at 37°C, and incubated overnight until the cells grew to confluency the next day.

### Step 2: Preparation of compounds

### Antagonist mode

1) Test compound stock solution concentration: 20 mM;
2) The compound on a 384-LDV plate was subjected to 12-point dilution using Bravo, starting from the concentration of 10 µM, with 3-fold dilutions;
3) HPE (high potency control): a single dose of positive control; FAC (final effective concentration): 40 µM; ZPE (zero potency control): 100% DMSO;
4) The compound on the 384-LDV plate as well as the HPE and the ZPE were transferred by ECHO to a 384-well plate (PE 6008590) as a compound plate;
5) The compound plate was stored at -20°C.

### Step 3: Screening

1) The plate with cells grown to confluency was removed from the incubator;
2) Assay buffer: a 30 mL buffer containing 0.3 mL 250 mM probenecid, 0.6 mL 1M HEPES, and 29.1 mL HBSS, with the actual amount of assay buffer depending on the number of cell plates;
3) C6 dye: C6 dye stock solution was 10×, diluted to 1× with the buffer;
4) The medium was discarded by inverted centrifugation using the gentle spin mode of Bluewasher.
5) C6 dye was added to the cell plate using a pipette displacement gun at 20 µL/well;
6) The cell plate was centrifuged at 300 rpm for 30 s and incubated in an incubator for 1.5 h;
7) 20 µL of the experimental buffer was added to each well in the prepared compound plate using a Dragonfly automated sample dispenser, 10 µL of the compound was transferred to the cell plate using Bravo according to the layout of the experimental plate, and the final assay concentration of the test compound at the highest dose FAC: 10 µM, 3-fold dilutions, 12 concentration points;
8) The cell plate was centrifuged at 300 rpm for 30 s and incubated in an incubator for 30 min;
9) 25 µL of 4×BZATP (final concentration 3.5 µ M) agonist was prepared on an agonist plate (PE 6008590) to act on P2X3 cells;
10) The cell plate, FLIPR gun tips and the agonist plate were placed at room temperature for 15 min;
11) 10 µL of BZATP agonist was transferred by FLIPR to the cell plate and read.

### Step 4: Experimental results and analysis

The IC50 of the inhibitory effect of the Example compounds against the P2X3 receptor was shown in the table below, wherein "A" denotes less than 10 nM and "B" denotes 10.1 to 100 nM.

**Table 4**

| Test sample | P2X3 IC50 (nM) | Test sample | P2X3 IC50 (nM) |
|---|---|---|---|
| Positive control | B | Example 6 | A |
| Comparative Example 1 | A | Example 7 | B |
| Example 1 | A | Example 8 | B |
| Example 2 | A | Example 9 | B |
| Example 3 | A | Example 10 | A |
| Example 4 | B | Example 11 | A |
| Example 5 | A | Example 12 | A |

The results show that several Example compounds of the present invention have an *in vitro* inhibitory effect on the P2X3 receptor superior to that of the positive control.

### Experimental Example 3: Taste disorder test

### 1 Test material

### 1.1 Basic information of the test material

The compounds of Examples 1, 3, 6, 11, 12 (synthesized in the laboratory by the present inventors), positive control (Gefapixant, Batch No.: 01030-210326-2-1, purchased from Tianjin Holder Pharmaceutical Technology Co., Ltd.).

### 1.2 Reagents

0.9% sodium chloride injection, quinine hydrochloride (Quinie, Batch No.: C12476271).

### 2 Laboratory animals

Healthy adult SD rats, all males, each weighing about 280 to 300 g.

### 3 Test methods

### 3.1 Preparation of the test material

Preparation of 0.3 mM quinine solution: 119.20 mg quinine hydrochloride was weighed and dissolved in 1,000 ml tap water, and mixed well.

Preparation of test sample solution: 40 mg of the test sample was weighed and added to an appropriate amount of DMSO and dissolved, then a solution of solubilizer HS-15 was added thereto and mixed well, and 16 ml saline was added, to obtain a solution of 2.5 mg/ml.

### 3.2 Experimental operations

Animals and grouping: male SD rats each about 160 to 180 g, 10 rats per group, with similar average weight in each group, fed in separate cages.

Drinking habit training: the animals in each group were given normal drinking water for 30 minutes at 8:30 am and 4:30 pm every day, with water deprivation for the rest of the day, and trained for 5 days, wherein two bottles of water placed in the left and right positions respectively were renewed every day.

Administration: After water deprivation over the night before the experiment, on the next morning 4 mL/kg (10 mg/kg) of the test sample was injected intravenously in the tail vein of the rats in the test group, and 4 mL/kg (10 mg/kg) of 0.5% HS-15 was injected intravenously in the model group.

### 4 Results and Discussion

### 4.1 Judgment criteria of results

(1) Drinking water measurement: after injection, the animals were put back to their original cages, and the measurement time of each group was in the window covering the Tₘₐₓ of the drugs (the measurement time was from 0 min to 15 min after administration); in each cage a bottle of normal drinking water and a bottle of drinking water containing 0.3 mM quinine hydrochloride (Quinie) were placed, and the left and right positions of the two bottles in all cages were the same. The animals were allowed to drink freely for 15 min, and the volume of water consumed from the two bottles was measured separately, accurate to 0.1 ml.
(2) Statistical analysis of data: the volume of the consumed quinine bitter water and the volume of the consumed tap water were measured, the percentage of quinine water relative to tap water was calculated, and ANOVA was used to check whether there was a significant difference between the groups.

### 4.2 Discussion

**Table 5**

| Test Samples | Quinine water/tap water (%) |
|---|---|
| Solvent group | 38.16% |
| Positive control group | 79.01%** |
| Example 1 | 37.01%^{△} |
| Example 3 | 38.11%^{△} |
| Example 6 | 41.54%^{△} |
| Example 11 | 37.12%^{△} |
| Example 12 | 39.39%^{△} |

| | |
|---|---|
| Note: as compared with the solvent group: **P<0.01; as compared with the positive control group: ^{△}P<0.01. | |

As can be seen through Table 5, the ratio of quinine water/tap water consumed by rats in the positive control group is statistically significantly different (P<0.01) from that of the solvent group, indicating that the positive control compound Gefapixant had a significant effect on the sense of taste of rats. In contrast, the ratio of quinine water/tap water consumed by rats in the groups of Examples 1, 3, 6, 11 and 12 did not show a statistically significant difference from that of the solvent group, indicating that the compounds of the present invention had almost no effect on the sense of taste of the rats after intravenous administration at 10 mg/kg. Moreover, the ratio of quinine water/tap water consumed by rats in the groups of Examples 1, 3, 6, 11 and 12 showed a statistically significant difference from that of the positive control group.

## Claims

1. A compound having a structure represented by Formula I, or a pharmaceutically acceptable salt thereof, 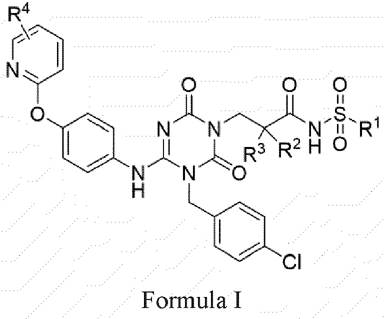
wherein R¹ is selected from a substituted or unsubstituted C1 to C12 alkyl, a substituted or unsubstituted C1 to C12 cycloalkyl, a substituted or unsubstituted C6 to C10 aryl, a substituted or unsubstituted C1 to C12 alkylamino group, and a substituted or unsubstituted C4 to C8 cycloalkylamino group;
R² and R³ are independently selected from hydrogen, halogen, a substituted or unsubstituted C1 to C12 alkyl, and a substituted or unsubstituted C1 to C12 cycloalkyl; or R² and R³ are joined together to form a substituted or unsubstituted 3-membered to 15-membered cycloalkyl group; and
R⁴ is selected from hydrogen or one or two of halogens, methyl, difluoromethyl, trifluoromethyl, difluoromethoxy, and trifluoromethoxy.

2. The compound of claim 1, or a pharmaceutically acceptable salt thereof,
wherein R² and R³ are independently selected from hydrogen, methyl, ethyl, propyl, isopropyl, and cyclopropyl;
or R² and R³ are joined together to form a cyclopropyl group, a cyclopentyl group, or a cyclohexyl group;
and/or R¹ is selected from a substituted or unsubstituted C1 to C6 alkyl, a substituted or unsubstituted C6 to C10 aryl, and a substituted or unsubstituted C1 to C6 alkylamino group;
wherein the substituents on R¹ are one or more selected from deuterium, halogen, hydroxyl, alkyl, alkenyl, alkynyl, alkoxy, alkylamino, alkylthio, amido, NO₂, CN, and CF₃.

3. The compound of claim 1, or a pharmaceutically acceptable salt thereof,
wherein R² and R³ are independently selected from hydrogen, methyl, ethyl, propyl, isopropyl, and cyclopropyl;
or R² and R³ are joined together to form a cyclopropyl group;
and/or R¹ is selected from methylamino, ethylamino, propylamino, isopropylamino, dimethylamino, tetrahydropyrrolyl, diethylamino, methyl, ethyl, propyl, isopropyl, trifluoromethyl, difluoromethyl, phenyl, halogenated phenyl, tolyl, halogenated tolyl, and halogenated methoxyphenyl.

4. The compound of claim 1, or a pharmaceutically acceptable salt thereof,
wherein R² and R³ are independently selected from hydrogen, methyl, and cyclopropyl; or R² and R³ are joined together to form a cyclopropyl group;
and/or R¹ is selected from methyl, ethyl, methylamino, dimethylamino, tetrahydropyrrolyl, diethylamino, trifluoromethyl, isopropylamino, phenyl, tolyl, and fluorophenyl.

5. The compound of claim 1, or a pharmaceutically acceptable salt thereof, which is selected from the compounds below or pharmaceutically acceptable salts thereof,

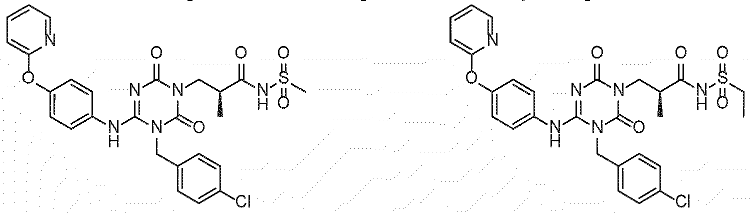

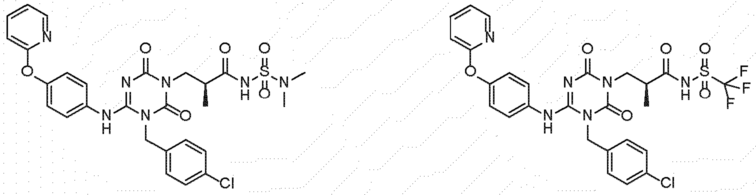

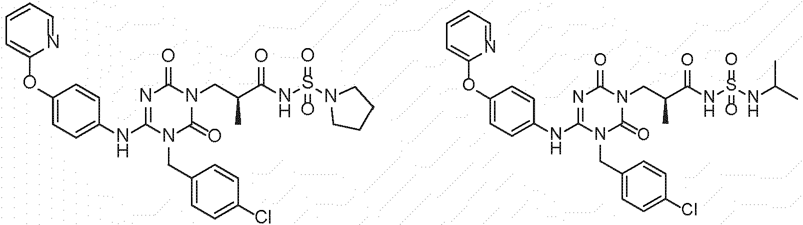

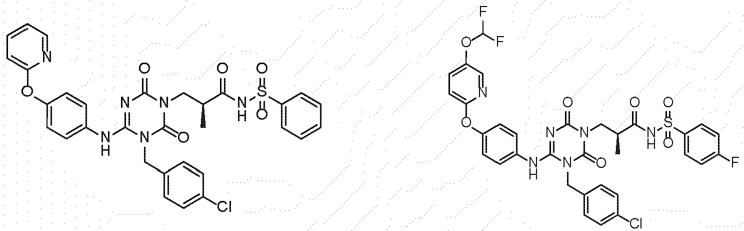

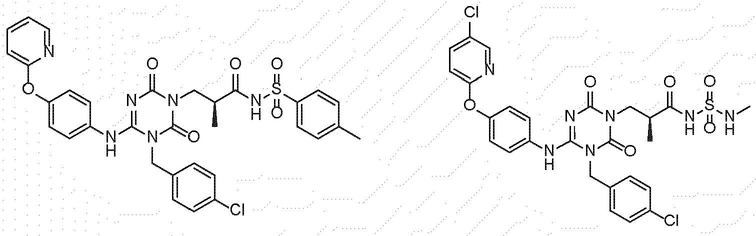

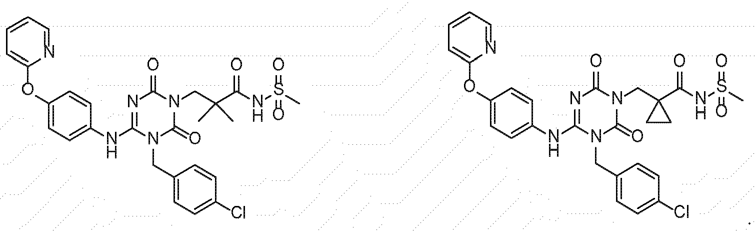

6. The compound of any one of claims 1 to 5, or a pharmaceutically acceptable salt thereof, wherein the hydrogen atoms in the compound are replaced by one or more deuterium atoms.

7. A method for preparing the compound of any one of claims 1 to 6 or a pharmaceutically acceptable salt thereof, comprising the following steps:
Step 1: allowing Compound **a** and Compound **k** to undergo a substitution reaction under a basic condition to produce Compound **b;**
Step 2: allowing intermediate Compounds **c** and **d** to undergo a substitution reaction under a basic condition to obtain Compound **e;**
Step 3: allowing intermediate Compounds **e** and **f** to undergo a Mitsunobu reaction to obtain Compound **g;**
Step 4: allowing intermediate Compound **g** and Compound **b** to undergo a coupling reaction to obtain Compound **h;**
Step 5: allowing Compound **h** to undergo a hydrolysis reaction catalyzed by an inorganic base or an acid to obtain Compound **j;**
Step 6: allowing Compound **j** and Compound **m** to undergo a condensation reaction to obtain a compound of Formula I; 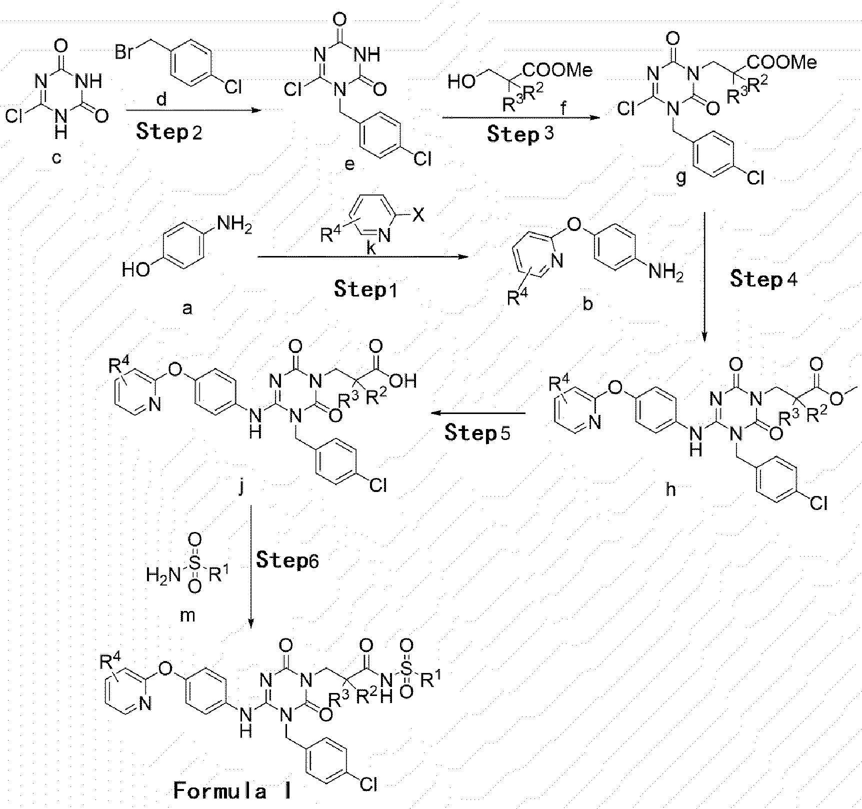 wherein R¹, R², R³, and R⁴ are defined as in claim 1, and X is a halogen.

8. A pharmaceutical formulation comprising the compound of any one of claims 1 to 6 and a pharmaceutically acceptable carrier.

9. The compound of any one of claims 1 to 6 or a pharmaceutically acceptable salt thereof for use in the treatment or prevention of P2X3 and/or P2X2/3 receptor-associated diseases.

10. The compound for use of claim 9, which is for use in the treatment or prevention of respiratory disorders, preferably in the treatment or prevention of cough, asthma, pain, or sleep apnea.

## Patentansprüche

1. Eine Verbindung, die eine durch Formel I dargestellte Struktur aufweist, oder ein pharmazeutisch annehmbares Salz davon, 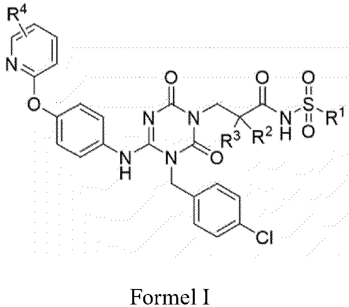
wobei R¹ aus einem substituierten oder unsubstituierten C1- bis C12-Alkyl, einem substituierten oder unsubstituierten C1- bis C12-Cycloalkyl, einem substituierten oder unsubstituierten C6- bis C10-Aryl, einer substituierten oder unsubstituierten C1- bis C12-Alkylaminogruppe und einer substituierten oder unsubstituierten C4- bis C8-Cycloalkylaminogruppe ausgewählt ist;
R² und R³ unabhängig voneinander aus Wasserstoff, Halogen, einem substituierten oder unsubstituierten C1- bis C12-Alkyl und einem substituierten oder unsubstituierten C1- bis C12-Cycloalkyl ausgewählt sind; oder R² und R³ miteinander verbunden sind, um eine substituierte oder unsubstituierte 3- bis 15-gliedrige Cycloalkylgruppe zu bilden; und
R⁴ aus Wasserstoff oder einem oder zwei Substituenten ausgewählt ist, die aus Halogen, Methyl, Difluormethyl, Trifluormethyl, Difluormethoxy und Trifluormethoxy ausgewählt sind.

2. Die Verbindung nach Anspruch 1 oder ein pharmazeutisch annehmbares Salz davon, wobei R² und R³ unabhängig voneinander aus Wasserstoff, Methyl, Ethyl, Propyl, Isopropyl und Cyclopropyl ausgewählt sind; oder R² und R³ miteinander verbunden sind, um eine Cyclopropylgruppe, eine Cyclopentylgruppe oder eine Cyclohexylgruppe zu bilden;
und/oder R¹ aus einem substituierten oder unsubstituierten C1- bis C6-Alkyl, einem substituierten oder unsubstituierten C6- bis C10-Aryl und einer substituierten oder unsubstituierten C1- bis C6-Alkylaminogruppe ausgewählt ist;
wobei die Substituenten an R¹ ein oder mehrere Substituenten sind, die aus Deuterium, Halogen, Hydroxyl, Alkyl, Alkenyl, Alkinyl, Alkoxy, Alkylamino, Alkylthio, Amido, NO₂, CN und CF₃ ausgewählt sind.

3. Die Verbindung nach Anspruch 1 oder ein pharmazeutisch annehmbares Salz davon, wobei R² und R³ unabhängig voneinander aus Wasserstoff, Methyl, Ethyl, Propyl, Isopropyl und Cyclopropyl ausgewählt sind;
oder R² und R³ miteinander verbunden sind, um eine Cyclopropylgruppe zu bilden;
und/oder R¹ aus Methylamino, Ethylamino, Propylamino, Isopropylamino, Dimethylamino, Tetrahydropyrrolyl, Diethylamino, Methyl, Ethyl, Propyl, Isopropyl, Trifluormethyl, Difluormethyl, Phenyl, halogeniertem Phenyl, Tolyl, halogeniertem Tolyl und halogeniertem Methoxyphenyl ausgewählt ist.

4. Die Verbindung nach Anspruch 1 oder ein pharmazeutisch annehmbares Salz davon, wobei R² und R³ unabhängig voneinander aus Wasserstoff, Methyl und Cyclopropyl ausgewählt sind; oder R² und R³ miteinander verbunden sind, um eine Cyclopropylgruppe zu bilden;
und/oder R¹ aus Methyl, Ethyl, Methylamino, Dimethylamino, Tetrahydropyrrolyl, Diethylamino, Trifluormethyl, Isopropylamino, Phenyl, Tolyl und Fluorphenyl ausgewählt ist.

5. Die Verbindung nach Anspruch 1 oder ein pharmazeutisch annehmbares Salz davon, ausgewählt aus den nachstehenden Verbindungen oder pharmazeutisch annehmbaren Salzen davon,

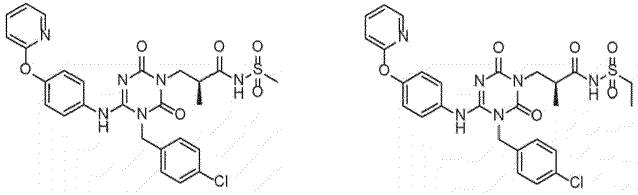

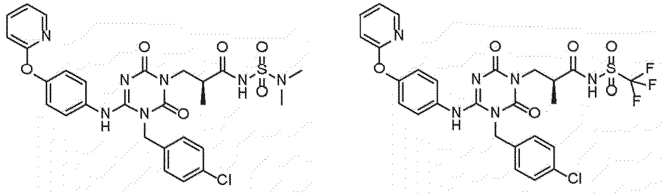

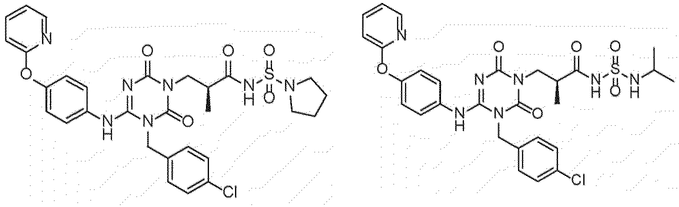

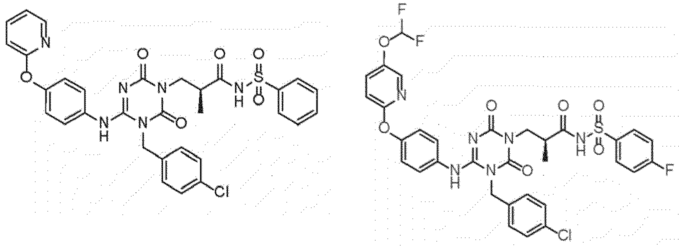

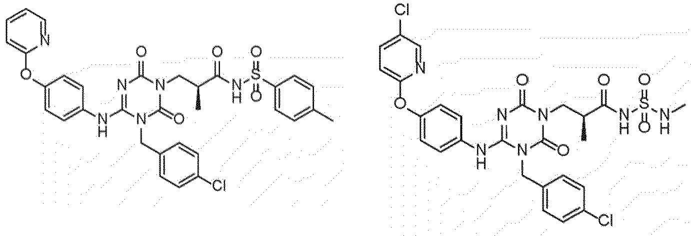

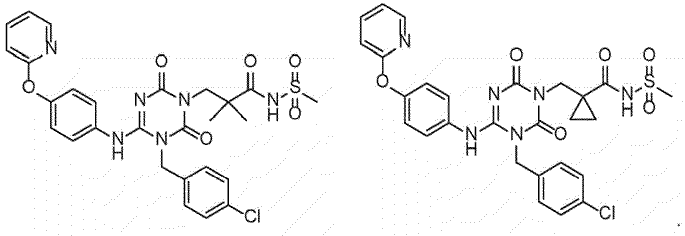

6. Die Verbindung nach einem der Ansprüche 1 bis 5 oder ein pharmazeutisch annehmbares Salz davon, wobei die Wasserstoffatome in der Verbindung durch ein oder mehrere Deuteriumatome ersetzt sind.

7. Ein Verfahren zur Herstellung der Verbindung nach einem der Ansprüche 1 bis 6 oder eines pharmazeutisch annehmbaren Salzes davon, umfassend die folgenden Schritte:
Schritt 1: Verbindung **a** und Verbindung **k** werden unter basischen Bedingungen einer Substitutionsreaktion unterzogen, um Verbindung b herzustellen;
Schritt 2: Die Zwischenverbindungen **c** und **d** werden unter basischen Bedingungen einer Substitutionsreaktion unterzogen, um Verbindung e zu erhalten;
Schritt 3: Die Zwischenverbindungen **e** und **f** werden einer Mitsunobu-Reaktion unterzogen, um Verbindung g zu erhalten;
Schritt 4: Die Zwischenverbindung **g** und Verbindung **b** werden einer Kupplungsreaktion unterzogen, um Verbindung h zu erhalten;
Schritt 5: Verbindung h wird einer durch eine anorganische Base oder eine Säure katalysierten Hydrolysereaktion unterzogen, um Verbindung **j** zu erhalten;
Schritt 6: Verbindung **j** und Verbindung **m** werden einer Kondensationsreaktion unterzogen, um eine Verbindung der Formel I zu erhalten; 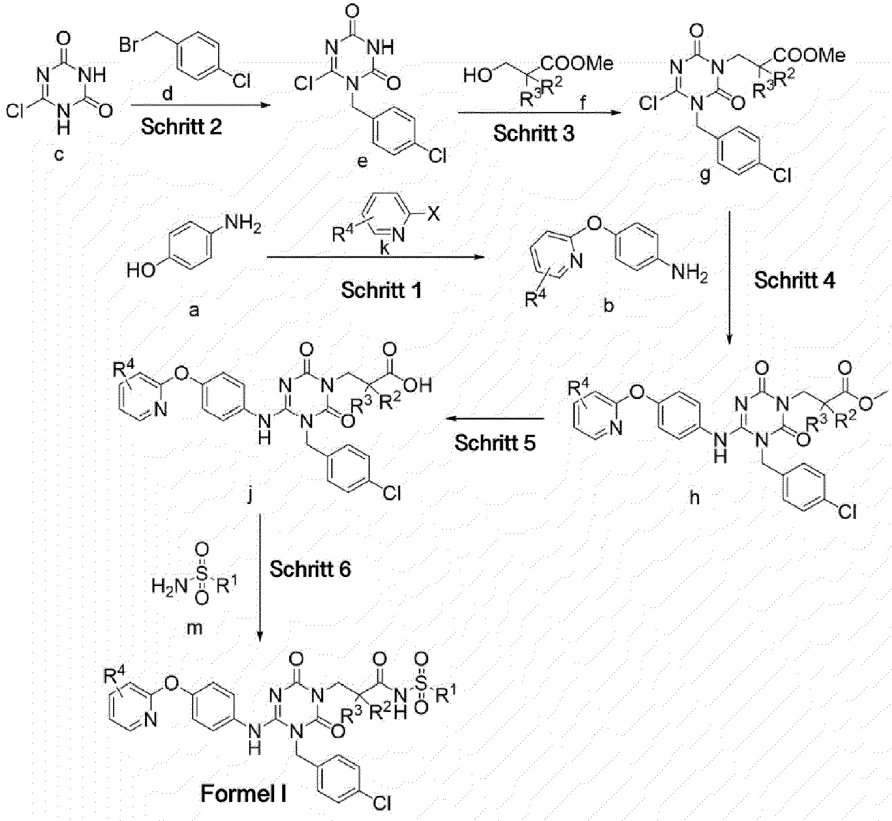 wobei R¹, R², R³ und R⁴ wie in Anspruch 1 definiert sind und X ein Halogen ist.

8. Eine pharmazeutische Formulierung, umfassend die Verbindung nach einem der Ansprüche 1 bis 6 und einen pharmazeutisch annehmbaren Träger.

9. Die Verbindung nach einem der Ansprüche 1 bis 6 oder ein pharmazeutisch annehmbares Salz davon zur Verwendung bei der Behandlung oder Vorbeugung von Erkrankungen, die mit P2X3- und/oder P2X2/3-Rezeptoren assoziiert sind.

10. Die Verbindung zur Verwendung nach Anspruch 9, die zur Verwendung bei der Behandlung oder Vorbeugung von Respiratorische Störungen, vorzugsweise bei der Behandlung oder Vorbeugung von Husten, Asthma, Schmerzen oder Schlafapnoe, bestimmt ist.

## Revendications

1. Un composé ayant une structure représentée par la formule I, ou un sel pharmaceutiquement acceptable dudit composé, 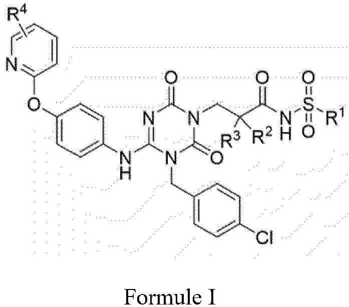
dans lequel R¹ est choisi parmi un alkyle en C1 à C12 substitué ou non substitué, un cycloalkyle en C1 à C12 substitué ou non substitué, un aryle en C6 à C10 substitué ou non substitué, un groupe alkylamino en C1 à C12 substitué ou non substitué, et un groupe cycloalkylamino en C4 à C8 substitué ou non substitué ;
R² et R³ sont indépendamment choisis parmi l'hydrogène, un halogène, un alkyle en C1 à C12 substitué ou non substitué, et un cycloalkyle en C1 à C12 substitué ou non substitué ; ou R² et R³ sont liés l'un à l'autre pour former un groupe cycloalkyle de 3 à 15 chaînons substitué ou non substitué ; et
R⁴ est choisi parmi l'hydrogène ou un ou deux substituants choisis parmi les halogènes, le méthyle, le difluorométhyle, le trifluorométhyle, le difluorométhoxy et le trifluorométhoxy.

2. Le composé selon la revendication 1, ou un sel pharmaceutiquement acceptable dudit composé, dans lequel R² et R³ sont indépendamment choisis parmi l'hydrogène, le méthyle, l'éthyle, le propyle, l'isopropyle et le cyclopropyle ; ou R² et R³ sont liés l'un à l'autre pour former un groupe cyclopropyle, un groupe cyclopentyle ou un groupe cyclohexyle ;
et/ou R¹ est choisi parmi un alkyle en C1 à C6 substitué ou non substitué, un aryle en C6 à C10 substitué ou non substitué et un groupe alkylamino en C1 à C6 substitué ou non substitué ;
dans lequel les substituants de R¹ sont un ou plusieurs substituants choisis parmi le deutérium, un halogène, un groupe hydroxyle, un groupe alkyle, un groupe alcényle, un groupe alcynyle, un groupe alcoxy, un groupe alkylamino, un groupe alkylthio, un groupe amido, NO₂, CN et CF₃.

3. Le composé selon la revendication 1, ou un sel pharmaceutiquement acceptable dudit composé, dans lequel R² et R³ sont indépendamment choisis parmi l'hydrogène, le méthyle, l'éthyle, le propyle, l'isopropyle et le cyclopropyle ;
ou R² et R³ sont liés l'un à l'autre pour former un groupe cyclopropyle ;
et/ou R¹ est choisi parmi le méthylamino, l'éthylamino, le propylamino, l'isopropylamino, le diméthylamino, le tétrahydropyrrolyle, le diéthylamino, le méthyle, l'éthyle, le propyle, l'isopropyle, le trifluorométhyle, le difluorométhyle, le phényle, le phényle halogéné, le tolyle, le tolyle halogéné et le méthoxyphényle halogéné.

4. Le composé selon la revendication 1, ou un sel pharmaceutiquement acceptable dudit composé, dans lequel R² et R³ sont indépendamment choisis parmi l'hydrogène, le méthyle et le cyclopropyle ; ou R² et R³ sont liés l'un à l'autre pour former un groupe cyclopropyle ;
et/ou R¹ est choisi parmi le méthyle, l'éthyle, le méthylamino, le diméthylamino, le tétrahydropyrrolyle, le diéthylamino, le trifluorométhyle, l'isopropylamino, le phényle, le tolyle et le fluorophényle.

5. Le composé selon la revendication 1, ou un sel pharmaceutiquement acceptable dudit composé, choisi parmi les composés ci-dessous ou leurs sels pharmaceutiquement acceptables,

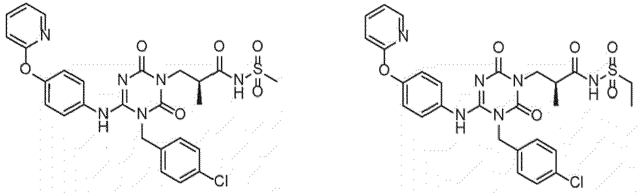

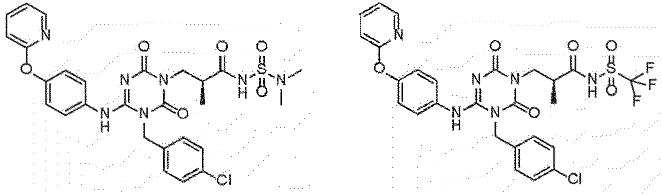

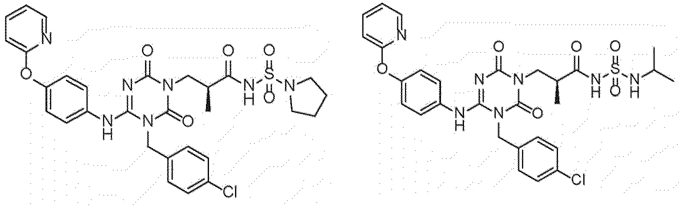

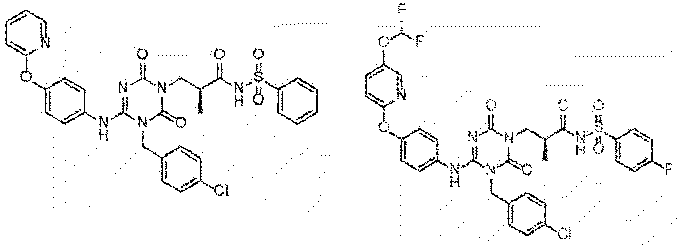

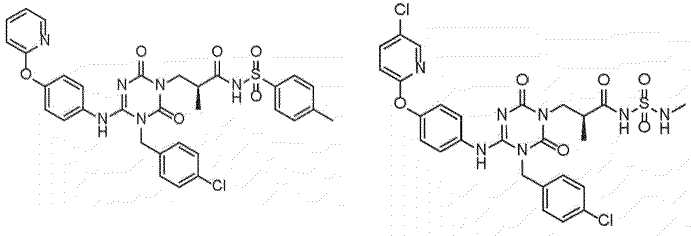

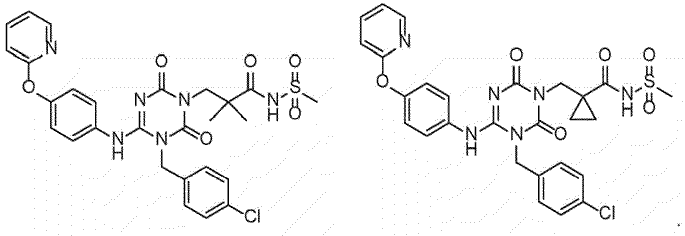

6. Le composé selon l'une quelconque des revendications 1 à 5, ou un sel pharmaceutiquement acceptable dudit composé, dans lequel les atomes d'hydrogène du composé sont remplacés par un ou plusieurs atomes de deutérium.

7. Un procédé de préparation du composé selon l'une quelconque des revendications 1 à 6 ou d'un sel pharmaceutiquement acceptable dudit composé, comprenant les étapes suivantes :
Étape 1 : soumettre les composés **a** et **k** à une réaction de substitution dans des conditions basiques pour produire le composé **b** ;
Étape 2 : soumettre les composés intermédiaires **c** et **d** à une réaction de substitution dans des conditions basiques pour obtenir le composé **e** ;
Étape 3 : soumettre les composés intermédiaires **e** et **f** à une réaction de Mitsunobu pour obtenir le composé **g** ;
Étape 4 : soumettre le composé intermédiaire **g** et le composé **b** à une réaction de couplage pour obtenir le composé **h** ;
Étape 5 : soumettre le composé h à une réaction d'hydrolyse catalysée par une base inorganique ou un acide pour obtenir le composé **j** ;
Étape 6 : soumettre les composés **j** et **m** à une réaction de condensation pour obtenir un composé de formule I ; 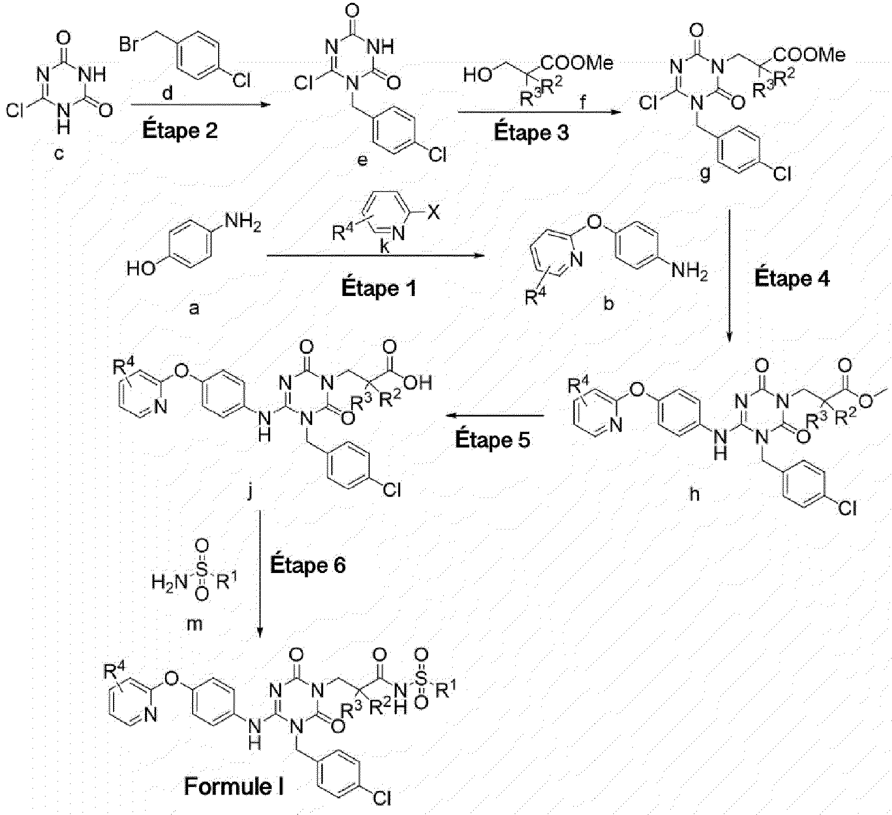 dans lequel R¹, R², R³ et R⁴ sont définis comme dans la revendication 1, et X est un halogène.

8. Une formulation pharmaceutique comprenant le composé selon l'une quelconque des revendications 1 à 6 et un véhicule pharmaceutiquement acceptable.

9. Le composé selon l'une quelconque des revendications 1 à 6 ou un sel pharmaceutiquement acceptable dudit composé, pour une utilisation dans le traitement ou la prévention de maladies associées aux récepteurs P2X3 et/ou P2X2/3.

10. Le composé pour une utilisation selon la revendication 9, lequel est destiné à être utilisé dans le traitement ou la prévention de troubles respiratoires, de préférence dans le traitement ou la prévention de la toux, de l'asthme, de la douleur ou de l'apnée du sommeil.
